(19) Europäisches Patentamt
European Patent Office
Office européen des brevets

(11) **EP 1 860 091 A1**

(12) **EUROPÄISCHE PATENTANMELDUNG**

(43) Veröffentlichungstag:
**28.11.2007 Patentblatt 2007/48**

(51) Int Cl.:
***C07C 51/265*** *(2006.01)*  ***C07C 51/31*** *(2006.01)*
***B01J 21/06*** *(2006.01)*

(21) Anmeldenummer: **06010681.2**

(22) Anmeldetag: **23.05.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **SÜD-CHEMIE AG**
**80333 München (DE)**

(72) Erfinder:
• **Gückel, Christian, Dr.**
  **Paramus NJ 07652 (US)**

• **Mestl, Gerhard, Dr.**
  **80935 München (DE)**
• **Estenfelder, Marvin, Dr.**
  **85560 Ebersberg (DE)**

(74) Vertreter: **Westendorp, Michael Oliver**
**Patentanwälte**
**Splanemann Reitzner**
**Baronetzky Westendorp**
**Rumfordstrasse 7**
**80469 München (DE)**

(54) **Katalysator enthaltend Titandioxid, insbesondere zur Herstellung von Phthalsäurenanhydrid**

(57)    Die vorliegende Erfindung beschreibt einen Katalysator, insbesondere zur Gasphasenoxidation von o-Xylol und/oder Naphthalin, mit einer katalytisch aktiven Masse enthaltend $TiO_2$ wobei das $TiO_2$ einen Gehalt an Schwefel, berechnet als elementarer Schwefel, von weniger als etwa 1000 ppm und eine BET-Oberfläche von mindestens 5 $m^2/g$ aufweist. Weiterhin wird ein bevorzugtes Verfahren zur Herstellung eines solchen Katalysators und dessen bevorzugte Verwendung beschrieben.

EP 1 860 091 A1

**Beschreibung**

Die Erfindung betrifft einen Katalysator enthaltend Titandioxid, insbesondere zur Herstellung von Phthalsäureanhydrid (PSA) durch Gasphasenoxidation von o-Xylol und/oder Naphthalin.

Die großtechnische Produktion von Phthalsäureanhydrid wird durch die katalytische Gasphasenoxidation von o-Xylol und/oder Naphthalin erzielt. Zu diesem Zwecke wird ein für die Reaktion geeigneter Katalysator in einen Reaktor, vorzugsweise einen sogenannten Rohrbündelreaktor, in dem eine Vielzahl von Rohren parallel angeordnet sind, gefüllt und von oben oder unten mit einem Gemisch aus dem (den) Kohlenwasserstoff(en) und einem sauerstoffhaltigen Gas, beispielsweise Luft, durchströmt. Aufgrund der starken Wärmebildung solcher Oxidationsreaktionen ist es nötig, die Reaktionsrohre zur Vermeidung von sogenannten Hot Spots ("Heißen Flecken") mit einem Wärmeträgermedium zu umspülen und somit die entstandene Wärmemenge abzuführen. Diese Energie kann zur Produktion von Dampf genutzt werden. Als Wärmeträgermedium dient in der Regel eine Salzschmelze und hier vorzugsweise ein eutektisches Gemisch aus $NaNO_2$ und $KNO_3$.

Ebenso kann man zur Unterdrückung der ungewollten Hot Spots einen strukturierten Katalysator in das Reaktionsrohr füllen, wodurch sich beispielsweise zwei oder drei Katalysatorlagen aus unterschiedlich zusammengesetzten Katalysatoren ergeben können. Solche Systeme sind als solche bereits aus der EP 1 082 317 B1 oder der EP 1 084 115 B1 bekannt.

Die schichtweise Anordnung der Katalysatoren hat auch den Zweck, den Gehalt an unerwünschten Nebenprodukten, d.h. Verbindungen, die in einem möglichen Reaktionsmechanismus von o-Xylol und/oder Naphthalin zu Phthalsäureanhydrid vor dem eigentlichen Wertprodukt stehen, im Roh-PSA so gering wie möglich zu halten. Zu diesen unerwünschten Nebenprodukten zählen hauptsächlich die Verbindungen o-Tolylaldehyd und Phthalid. Die Weiteroxidation dieser Verbindungen zu Phthalsäureanhydrid steigert zudem die Selektivität bzgl. des eigentlichen Wertprodukts.

Neben den oben angesprochenen Unteroxidationsprodukten treten bei der Reaktion auch Überoxidationsprodukte auf. Dazu gehören Maleinsäureanhydrid, Citraconsäureanhydrid, Benzoesäure und die Kohlenoxide. Eine gezielte Unterdrückung der Bildung dieser ungewünschten Nebenprodukte zu Gunsten des Wertprodukts führt zu einem weiteren Anstieg der Produktivität und Wirtschaftlichkeit des Katalysators.

Entsprechende Überlegungen gelten auch bei anderen Katalysatoren, z.B. zur partiellen Oxidation anderer Kohlenwasserstoffe.

Es besteht ein ständiger Bedarf an Katalysatoren, die einen hohen Umsatz bei hoher Selektivität aufweisen, und somit eine verbesserte Produktivität und Wirtschaftlichkeit ermöglichen.

Eine Aufgabe der vorliegenden Erfindung war es daher, einen Katalysator bzw. ein Katalysatorsystem zu entwickeln, das die Nachteile bekannter Katalysatoren aus dem Stand der Technik vermeidet und eine Verbesserung der Aktivität, Selektivität und/oder Lebensdauer des Katalysators ermöglicht.

Diese Aufgabe wird durch einen Katalysator gemäß Anspruch 1 gelöst. Demnach betrifft ein erster Aspekt der Erfindung einen Katalysator, insbesondere zur Gasphasenoxidation von O-Xylol und/oder Naphthalin, mit einer katalytisch aktiven Masse enthaltend $TiO_2$, wobei das $TiO_2$ einen Gehalt an Schwefel, berechnet als elementarer Schwefel, von weniger als etwa 1000 ppm und vorzugsweise eine BET-Oberfläche von mindestens 5 $m^2/g$ aufweist.

So wurde im Rahmen der vorliegenden Erfindung überraschend gefunden, dass die Verwendung von $TiO_2$ mit einem Gehalt an Schwefel, berechnet als elementarer Schwefel von weniger als 1000 ppm zu verbesserten Katalysatoren führt, die z.B. bei der Gasphasenoxidation von o-Xylol und/oder Naphthalin zu Phthalsäureanhydrid eine verbesserte $C_8$-Selektivität und eine vorteilhafte geringe $CO_x$-Selektivität des Katalysators bei gleichzeitig verbessertem Umsatz ermöglichen. Zugunsten einer verbesserten PSA-Selektivität wurde auch unerwartet weniger MSA (Maleinsäureanhydrid) als Nebenprodukt gebildet.

Weiter bevorzugt liegt dabei der Gehalt des eingesetzten $TiO_2$ an Schwefel (berechnet als elementarer Schwefel) bei weniger als etwa 900 ppm, insbesondere weniger 750 ppm, bevorzugt weniger als 500 ppm, weiter bevorzugt weniger als etwa 300 ppm.

Im Rahmen der vorliegenden Erfindung wurde weiterhin gefunden, dass sich die Vorteile des erfindungsgemäßen Katalysators, enthalten $TiO_2$ mit geringer Verunreinigung an Schwefel besonders deutlich zeigen, wenn das $TiO_2$ eine BET-Oberfläche von mindestens 5 $m^2/g$ , insbesondere von mindestens 12 $m^2/g$ aufweist. Bei der bevorzugten Verwendung des Katalysators zur Gasphasenoxidation von o-Xylol und/oder Naphthalin zu Phthalsäureanhydrid liegt die BET-Oberfläche (DIN 66131) des verwendeten $TiO_2$-Materials vorzugsweise im Bereich zwischen etwa 15 und 60 $m^2/g$, insbesondere zwischen 15 und 45 $m^2/g$, weiter bevorzugt zwischen 15 und 35 $m^2/g$.

Es wurde nach einem weiteren Aspekt der vorliegenden Erfindung auch unerwartet gefunden, dass ein geringer Gehalt des verwendeten $TiO_2$ an Phosphor, berechnet als elementarer Phosphor, eine besonders vorteilhafte Selektivität des Katalysators bei sehr gutem Umsatz ermöglicht. Demnach weist nach einer bevorzugten erfindungsgemäßen Ausführungsform das verwendete $TiO_2$ einen Gehalt an Phosphor, berechnet als elementarer Phosphor, von weniger als etwa 800 ppm, bevorzugt von weniger als etwa 700 ppm, insbesondere weniger als etwa 500 ppm, insbesondere von weniger als etwa 300 ppm auf. Im Falle der Herstellung von Phthalsäureanhydrid wurde dabei auch zugunsten einer verbesserten PSA-Selektivität unerwartet weniger MSA (Maleinsäureanhydrid) als Nebenprodukt gebildet. Besonders bevorzugt weist das erfindungsgemäß verwendete $TiO_2$ sowohl den geringen Schwefelgehalt als auch den geringen P-Gehalt wie vorstehend definiert auf. Nach einem weiteren Aspekt der vorliegenden Erfindung wurde jedoch gefunden, dass auch $TiO_2$-Materialien, die den vorstehenden geringen P-Gehalt aufweisen, selbst bei einem höheren S-Gehalt (mehr als etwa 1000 ppm) eine bessere Aktivität und Selektivität zeigen als $TiO_2$-Materialien, die den vorstehenden geringen P-Gehalt nicht aufweisen.

Erfindungsgemäß weist zumindest ein Teil des in dem Katalysator verwendeten $TiO_2$ die vorstehende Spezifikation im Hinblick auf den Schwefelgehalt und vorzugsweise auch den Phosphorgehalt auf. Bevorzugt wird der erfindungsgemäße Katalysator jedoch überwiegend, d.h. zu mehr als 50%, insbesondere mehr als 75%, weiter bevorzugt mehr als 90%, insbesondere im Wesentlichen oder vollständig nur $TiO_2$-Material mit der vorstehenden Spezifikation enthalten. Es können auch Abmischungen verschiedener $TiO_2$-Materialien verwendet werden.

Geeignete $TiO_2$-Materialien sind kommerziell erhältlich oder können vom Fachmann nach Standard-Verfahren erhalten werden, soweit bei der Synthese darauf geachtet wird, dass die verwendeten Ausgangsreagenzien bzw. Rohstoffe entsprechend geringe Verunreinigungen an Schwefel bzw. Phosphor enthalten. Alternativ kann auch von $TiO_2$-Materialien mit höherem S- bzw. P-Gehalt ausgegangen werden und durch geeignetes Waschen der erfindungsgemäß vorausgesetzte Bereich eingestellt werden. Beispielsweise kann in abfolgenden Waschschritten mit 0,1 - 1 molarer Salpetersäure, bidestilliertem Wasser, 1 molarem Ammoniakwasser und abschließend erneut mit bidestilliertem Wasser gewaschen werden. Dieser Waschzyklus kann im Bedarfsfall auch ein- oder mehrmals wiederholt werden. Die Dauer der einzelnen Waschschritte kann auch variiert werden. Beispielsweise kann ein Waschschritt für 3 bis 16 Stunden durchgeführt werden. Nach jedem Waschschritt kann das Material auf herkömmliche Weise vor dem nächsten Waschschritt von der jeweiligen Waschlösung abgetrennt werden, beispielsweise durch Filtration. Nach dem letzten Waschschritt kann das Material getrocknet werden.

Nach einer weiter bevorzugten erfindungsgemäßen Ausführungsform liegt der Gehalt an Niob (berechnet als Nb) des verwendeten $TiO_2$ bei mehr als etwa 500 ppm, insbesondere mehr als 1000 ppm. So wurde gefunden, dass dabei eine hohe Aktivität des Katalysators, insbesondere bei der Gasphasenoxidation von o-Xylol und/oder Naphthalin zu Phthalsäureanhydrid bei sehr hoher $C_8$-Selektivität bzw. Phthalsäureanhydrid (PSA)-Selektivität erzielt werden kann. Der bevorzugte Gehalt an Niob lässt sich z.B. durch die Verwendung von Niobsäure oder Nioboxalat während der Herstellung des $TiO_2$ einstellen.

Ein Verfahren zur Bestimmung des Anteils der hierin genannten Verunreinigungen im $TiO_2$, insbesondere der S-, P- und Nb-Gehalte des eingesetzten $TiO_2$ ist nachstehend vor den Beispielen angegeben (DIN ISO 9964-3).

Nach einer weiteren bevorzugten Ausführungsform enthält die Aktivmasse (katalytisch aktive Masse) des erfindungsgemäßen Katalysators Titandioxid mit einer spezifischen BET-Oberfläche und vorzugsweise einer spezifischen Porenradienverteilung, wozu auf die parallel WO 2005/11615 A1 der gleichen Anmelderin Bezug genommen wird. Danach wird die Verwendung eines Titandioxid bevorzugt, worin mindestens 25%, insbesondere mindestens etwa 40%, besonders bevorzugte mindestens etwa 50%, am meisten bevorzugt mindestens etwa 60% des gesamten Porenvolumens durch Poren mit einem Radius zwischen 60 und 400 nm gebildet werden. Weiterhin wird danach nach einer bevorzugten Ausführungsform ein $TiO_2$ verwendet, das eine Primärkristallitgröße (Primärpartikelgröße) von mehr als etwa 210 Angström, vorzugsweise mehr als etwa 250 Angström, weiter bevorzugt mindestens 300 Angström, insbesondere mindestens etwa 350 Angström, weiter bevorzugt mindestens 390 Angström aufweist. So wurde gefunden, dass solche $TiO_2$-Primärkristallite mit der vorstehenden (Mindest-)Größe die Herstellung von besonders vorteilhaften Katalysatoren ermöglichen. Bevorzugt liegt die Primärkristallitgröße unter 900 Angström, insbesondere unter 600 Angström, weiter bevorzugt unter 500 Angström. Die vorstehende Primärkristallitgröße ermöglicht offenbar, ohne dass die Erfindung auf diese Annahme beschränkt wäre, die Ausbildung einer nicht zu kompakten, sondern offenporigen Struktur des Titandioxids im Katalysator. Ein Verfahren zur Bestimmung der Primärkristallitgröße ist im nachstehenden Methodenteil angegeben.

Nach einer weiteren bevorzugten Ausführungsform wird $TiO_2$ verwendet, das eine Schüttdichte von weniger als 1,0 g/ml, insbesondere weniger als 0,8 g/ml, besonders bevorzugt weniger als etwa 0,6 g/ml aufweist. Am meisten bevorzugt sind $TiO_2$-Materialien mit einer Schüttdichte von nicht mehr als etwa 0,55 g/ml. Ein Verfahren zur Bestimmung der Schüttdichte ist im nachstehenden Methodenteil angegeben. Es wurde somit gefunden, dass die Verwendung eines Titandioxids mit einer Schüttdichte wie vorstehend definiert, die Herstellung besonders leistungsfähiger Katalysatoren ermöglicht. Es wird angenommen, ohne dass die Erfindung hierauf beschränkt wäre, dass die Schüttdichte hier ein Maß für eine besonders günstige Struktur der im Katalysator zur Verfügung gestellten $TiO_2$-Oberfläche ist, wobei durch die lockere, nicht zu kompakte Struktur besonders günstige Reaktionsräume sowie Zuführ- und Ableitwege für die Reaktanden bzw. Reaktionsprodukte bereitgestellt werden.

Die erfindungsgemäßen Katalysatoren können bei verschiedenen Umsetzungen vorteilhaft eingesetzt werden. Nach einer bevorzugten Ausführungsform der vorliegenden Erfindung wird der $TiO_2$-haltige Katalysator dabei zur Gasphasenoxidation von Kohlenwasserstoffen eingesetzt. Insbesondere bevorzugt ist die Verwendung zur Herstellung von Phthalsäureanhydrid durch Gasphasenoxidation von o-Xylol, Naphthalin oder Gemischen davon. Es sind jedoch auch eine Vielzahl von anderen katalytischen Gasphasenoxidationen von aromatischen Kohlenwasserstoffen wie Benzol, Xylolen, Naphthalin, Toluol oder Durol zur Herstellung von Carbonsäuren und/oder Carbonsäureanhydriden im Stand der Technik bekannt. Dabei werden beispielsweise Benzoesäure, Maleinsäureanhydrid, Isophthalsäure, Terephthalsäure oder Pyromellithsäureanhydrid gewonnen. Auch bei solchen Umsetzungen kann der erfindungsgemäße Katalysator eingesetzt werden.

Auch bei der partiellen Oxidation von Alkoholen zu den entsprechenden Aldehyden oder / und Carbonsäuren, wie beispielsweise der Oxidation von Methanol zu Formaldehyd, oder Carbonsäuren oder / und der Oxidation von Aldehyden zu den entsprechenden Carbonsäuren, ist die Verwendung des erfindungsgemäßen Katalysators von Vorteil.

Von Interesse ist beispielsweise auch die Verwendung bei der Ammoxidation von Alkanen und Alkenen, der Ammoxidation von Alklyaromaten und Alkylheteroaromaten zu den entsprechenden Cyanoverbindungen, insbesondere die Ammoxidation von 3-Methylpyridin (b-Picolin) zu 3-Cyanopyridin, bei der Oxidation von 3-Methylpyridin zu Nicotinsäure, bei der Oxidation von Acenaphthen zu Naphthalsäureanhydrid, oder bei der Oxidation von Duren zu Pyromellitsäureanhydrid. Eine bevorzugte Verwendung umfasst auch die Herstellung von Naphthalsäureanhydrid aus Acenaphthen sowie die Herstellung von Cyanopyridin aus Alkylpyridin (Picolin) durch Ammoxidation, wie beispielsweise von 3-Methylpyridin zu 3-Cyanopyridin. Beispiele für die allgemeine Zusammensetzung hierzu geeigneter Katalysatoren und Reaktionsbedingungen finden sich beispielsweise in Saurambaeva und Sembaev, Eurasian ChemTech Journal 5 (2003), S, 267 - 270. Eine Übersicht zur Amm(oxidation) von Methylpyridinen findet sich beispielsweise in R. Chuck, Applied Catalysis, A: General (2005), 280(1), 75-82. Weitere vorteilhafte Verwendungen des erfindungsgemäßen Katalysators bzw. des $TiO_2$ wie hierin definiert betreffen oxidative Dehydrierungen, beispielsweise von Ethan, Propan, Butan, Isobutan oder längerkettiger Alkane zu den jeweiligen Alkenen.

Die erfindungsgemäßen Katalysatoren, insbesondere für die oben beschriebenen Ammoxidations- und Oxidationsreaktionen, können Vollkatalysatoren oder Schalenkatalysatoren in Form der dem Fachmann bekannten Formkörper und Geometrien sein. Besonders vorteilhaft ist es, wenn die aktive Masse auf einen inerten Träger aufgebracht ist.

Im Allgemeinen wird bei der Umsetzung ein Gemisch aus einem molekularen Sauerstoff enthaltenden Gas, beispielsweise Luft, und dem zu oxidierenden Ausgangsmaterial durch einen Festbettreaktor, insbesondere einen Rohrbündelreaktor, der aus einer Vielzahl parallel angeordneter Rohre bestehen kann, geleitet. In den Reaktorrohren befindet sich jeweils eine Schüttung aus mindestens einem Katalysator. Häufig ist eine Schüttung aus mehreren (unterschiedlichen) Katalysatorlagen vorteilhaft.

Beim Einsatz der erfindungsgemäßen Katalysatoren zur Herstellung von Phthalsäureanhydrid durch Gasphasenoxidation von o-Xylol und/oder Naphthalin wurde überraschend festgestellt, dass die erfindungsgemäßen Katalysatoren einen hohen Umsatz bei gleichzeitig geringer Bildung der unerwünschten Nebenprodukte $CO_x$, d.h. $CO_2$ und CO, erhalten wird. Des weiteren zeigen sich eine sehr gute $C_8$- und PSA-Selektivitäten, wodurch in Summe die Produktivität des Katalysators erhöht wird. Durch die geringe $CO_x$-Selektivität ergibt sich auch in vorteilhafter Weise eine geringere Wärmeentwicklung sowie niedrigere Hot Spot-Temperaturen. Dadurch kommt es zu einer langsameren Deaktivierung des Katalysators im Hot Spot-Bereich.

Die Bestimmung der hierin angegebenen Porenvolumina bzw. -anteile erfolgt, soweit nicht anders angegeben, mittels Quecksilberporosimetrie (gemäß DIN 66133). Die Angabe des Gesamtporenvolumens bezieht sich dabei in der vorliegenden Beschreibung jeweils auf das gesamte mittels Quecksilberporosimetrie gemessene Porenvolumen zwischen 7500 und 3,7 nm Porenradiengröße.

Nach einer weiteren bevorzugten Ausführungsform weist das eingesetzte $TiO_2$ die folgende Teilchengrößenverteilung auf: der $D_{10}$-Wert liegt vorzugsweise bei 0,5 $\mu$m oder darunter; der $D_{50}$-Wert (d.h. der Wert, bei dem jeweils die Hälfte der Teilchen einen größeren bzw. kleineren Teilchendurchmesser aufweist) liegt vorzugsweise bei 1,5 $\mu$m oder darunter; der $D_{90}$-Wert liegt vorzugsweise bei 4 $\mu$m oder darunter. Bevorzugt liegt der $D_{90}$-Wert des eingesetzten $TiO_2$ zwischen etwa 0,5 und 20 $\mu$m, insbesondere zwischen etwa 1 und 10 $\mu$m, besonders bevorzugt zwischen etwa 2 und 5 $\mu$m. Das Titandioxid liegt vorzugsweise in der Anatasform vor.

Nach einer möglichen erfindungsgemäßen Ausführungsform kann auch nur ein Teil des zur Katalysatorherstellung verwendeten Titandioxids die hierin beschriebenen Eigenschaften aufweisen, obwohl dies in der Regel nicht bevorzugt ist. Auch die Form des Katalysators bzw. dessen homogener oder heterogener Aufbau ist im Sinne der vorliegenden Erfindung grundsätzlich nicht beschränkt und kann jegliche dem Fachmann geläufige und für das jeweilige Anwendungsgebiet geeignet erscheinende Ausführungsform umfassen.

In vielen Fällen, so auch wenn der erfindungsgemäße Katalysator nach einer besonders bevorzugten Ausführungsform zur Herstellung von Phthalsäureanhydrid eingesetzt wird, haben sich sogenannte Schalenkatalysatoren bewährt. Hierbei wird ein unter den Reaktionsbedingungen inerter Träger, beispielsweise aus Quarz ($SiO_2$), Porzellan, Magnesiumoxid, Zinndioxid, Siliciumcarbid, Rutil, Tonerde ($Al_2O_3$), Aluminiumsilicat, Magnesiumsilicat (Steatit), Zirkoniumsilicat oder Cersilicat, oder aus Mischungen der vorstehenden Materialien verwendet. Der Träger kann beispielsweise die Form von Ringen, Kugeln, Schalen oder Hohlzylindern aufweisen. Darauf wird in verhältnismäßig dünnen Schichten (Schalen) die katalytisch aktive Masse aufgebracht. Es können auch zwei oder mehrere Schichten der gleichen oder unterschiedlich zusammengesetzter katalytisch aktiver Masse aufgebracht werden.

Je nach vorgesehener Verwendung des erfindungsgemäßen Katalysators können neben dem erfindungsgemäß eingesetzten $TiO_2$ die dem Fachmann geläufigen und üblichen Komponenten in der aktiven Masse des Katalysators enthalten sein, wobei $TiO_2$ (einschließlich der hierin genannten Verunreinigungen) vorzugsweise etwa 40 bis 99 Gew.-% der aktiven Masse des Katalysators bildet. Bevorzugt enthalten die erfindungsgemäßen Katalysatoren neben $TiO_2$ noch Vanadiumoxid. Daneben sind ggf. noch Oxide von Niob und/oder Antimon und oder weitere Komponenten wie z.B. Cs und/oder P enthalten. Bezüglich der weiteren Komponenten der katalytisch aktiven Masse des erfindungsgemäßen Katalysators (neben $TiO_2$) kann grundsätzlich auf die im einschlägigen Stand der Technik beschriebenen und dem Fachmann geläufigen Zusammensetzungen bzw. Komponenten verwiesen werden. Dabei handelt es sich, wie vorstehend ausgeführt, hauptsächlich um Katalysatorsysteme, die neben Titanoxid(en) Oxide des Vanadiums enthalten. Beispielhafte Katalysatoren sind z.B. in der EP 0 964 744 B1 beschrieben, deren diesbezüglich Offenbarung hiermit ausdrücklich durch Inbezugnahme in die Beschreibung aufgenommen wird.

Nach einer bevorzugten erfindungsgemäßen Ausführungsform enthalten die erfindungemäßen Katalysatoren bzw. deren aktive Masse:

| | |
|---|---|
| $V_2O_5$ | 0 - 30 Gew.-%, insbesondere 1 - 30 Gew.-% |
| $Sb_2O_3$ oder $Sb_2O_5$ | 0 - 10 % Gew.-% |
| Cs | 0 - 2 % Gew.-% |
| P | 0 - 5 % Gew.-% |
| Nb | 0 - 5 % Gew.-% |
| Weitere Komponenten wie Ba, W, Mo, Y, Ce, Mg, Sn, Bi, Fe, Ag, Co, Ni, Cu, Au, Sn, Zr etc. | 0 - 5 % Gew.-% |
| $TiO_2$ (einschließlich der Verunreinigungen) | 40 bis 99 Gew.-%, insbesondere Rest zu 100 Gew-% |

Insbesondere sind im Stand der Technik eine Reihe von Promotoren zur Steigerung der Produktivität der Katalysatoren beschrieben, die im erfindungsgemäßen Katalysator ebenfalls eingesetzt werden können. Dazu gehören u.a. die Alkali- und Erdalkalimetalle, Thallium, Antimon, Phosphor, Eisen, Niob, Kobalt, Molybdän, Silber, Wolfram, Zinn, Blei, Zirkon, Kupfer, Gold und/oder Bismut sowie Mischungen aus zwei oder mehreren der vorstehenden Komponenten. Beispielsweise ist in der DE 21 59 441 A ein Katalysator beschrieben, der neben Titandioxid der Anatas-Modifikation aus 1 bis 30 Gew.-% Vanadiumpentoxid und Zirkondioxid besteht. Über die einzelnen Promotoren lässt sich die Aktivität und Selektivität der Katalysatoren beeinflussen, insbesondere durch Absenkung oder Erhöhung der Aktivität. Zu den die Selektivität erhöhenden Promotoren zählen beispielsweise die Alkalimetalloxide, wohingegen oxidische Phosphorverbindungen, insbesondere Phosphorpentoxid, die Aktivität des Katalysators auf Kosten der Selektivität erhöhen.

Im Rahmen der vorliegenden Erfindung wurde überraschend festgestellt, dass die Wirkung des im verwendeten $TiO_2$, gegebenenfalls nach der vorher beschriebenen Waschprozedur, vorhandenen Schwefels und/oder Phosphors eine andere ist als bei gesonderter Zugabe des Schwefels und/oder Phosphors während der Katalysatorsynthese (als zusätzliche Schwefel bzw. Phosphor enthaltende Komponente(n) des Katalysators außer den im $TiO_2$ vorhandenen Schwefel- und Phosphoranteilen). Die hierin gemachten Mengenangaben für solche zusätzlichen Schwefel bzw. Phosphor enthaltenden Komponenten des Katalysators umfassen daher nicht die S- bzw. P-Verunreinigung des verwendeten $TiO_2$. Es wird vermutet, ohne dass die Erfindung auf diese Annahme beschränkt ist, dass das im $TiO_2$ erfindungsgemäß als nur geringe Verunreinigung vorhandene S und/oder P stark an das $TiO_2$ gebunden ist bzw. sogar in das Gitter eingebaut ist. Die bei der Herstellung der erfindungsgemäßen Katalysatoren gegebenenfalls zugegebenen weiteren Schwefel und/oder Phosphor enthaltenden Komponenten sind offenbar nur zum Teil auf der Oberfläche des $TiO_2$ adsorbiert, während ein Großteil in Wechselwirkung mit den katalytisch aktiven Bestandteilen wie den Oxiden des Vanadiums oder anderen gegebenenfalls vorhandenen Oxiden treten kann. Entsprechendes gilt für Niob.

Zur Herstellung der erfindungsgemäßen Katalysatoren sind im Stand der Technik zahlreiche geeignete Verfahren beschrieben, so dass eine detaillierte Darstellung hier grundsätzlich nicht erforderlich ist. Es können alle üblichen und dem Fachmann geläufigen Katalysatorformen gewählt werden, einschließlich von Vollkatalysatoren und Schalenkatalysatoren, die einen inerten Träger und mindestens eine darauf aufgebrachte Schicht mit einer katalytisch aktiven Masse, enthaltend das erfindungsgemäß verwendete $TiO_2$, aufweisen. Zur Herstellung von Schalenkatalysatoren kann beispielsweise auf das in der DE-A-16 42 938 oder der DE-A 17 69 998 beschriebene Verfahren verwiesen werden, worin eine ein wässriges und/oder ein organisches Lösungsmittel enthaltende Lösung oder Suspension der Komponenten der katalytisch aktiven Masse und/oder deren Vorläuferverbindungen (häufig als "Maische" bezeichnet) auf das Trägermaterial in einer beheizten Dragiertrommel bei erhöhter Temperatur aufgesprüht werden, bis der gewünschte Gehalt an katalytisch aktiver Masse, bezogen auf das Katalysatorgesamtgewicht, erreicht ist.

Bevorzugt werden sogenannte Schalenkatalysatoren durch das Aufbringen einer dünnen Schicht von 50 bis 500 $\mu$m der Aktivkomponenten auf einen inerten Träger hergestellt (z.B. US 2,035,606). Als Träger haben sich insbesondere Kugeln oder Hohlzylinder bewährt. Diese Formkörper ergeben eine hohe Packungsdichte bei niedrigem Druckverlust und verringern die Gefahr der Bildung von Packungsfehlern beim Einfüllen des Katalysators in die Reaktionsrohre.

Die geschmolzenen und gesinterten Formkörper müssen innerhalb des Temperaturbereiches der ablaufenden Reaktion hitzebeständig sein. Wie vorstehend ausgeführt, kommen dabei beispielsweise Siliciumcarbid, Steatit, Quarz, Porzellan, $SiO_2$ $Al_2O_3$ oder Tonerde in Frage.

Der Vorteil der Beschichtung von Trägerkörpern im Wirbelbett ist die hohe Gleichmäßigkeit der Schichtdicke, die für die katalytische Leistung des Katalysators eine entscheidende Rolle spielt. Eine besonders gleichmäßige Beschichtung erhält man durch Aufsprühen einer Suspension oder Lösung der Aktivkomponenten auf den erwärmten Träger bei 80 bis 200°C im Wirbelbett, beispielsweise gemäß DE 12 80 756, DE 198 28 583 oder DE 197 09 589. Im Gegensatz zu der Beschichtung in Dragiertrommeln kann bei Verwendung von Hohlzylindern als Träger in den genannten Wirbelbettverfahren auch die Innenseite der Hohlzylinder gleichmäßig beschichtet werden. Unter den oben genannten Wirbelbettverfahren ist insbesondere das Verfahren nach DE 197 09 589 von Vorteil, da durch die überwiegend horizontale, kreisförmige Bewegung der Träger neben einer gleichmäßigen Beschichtung auch eine geringe Abrasion von Apparateteilen erreicht wird.

Für den Beschichtungsvorgang wird die wässrige Lösung oder Suspension der Aktivkomponenten und eines organischen Binders, vorzugsweise einem Copolymer aus Vinylacetat/Vinyllaurat, Vinylacetat/Ethylen oder Styrol/Acrylat, über eine oder mehrere Düsen auf den erwärmten, fluidisierten Träger aufgesprüht. Besonders günstig ist es, die Sprühflüssigkeit am Ort der höchsten Produktgeschwindigkeit aufzugeben, wodurch sich der Sprühstoff gleichmäßig im Bett verteilen kann. Der Sprühvorgang wird solange fortgeführt, bis entweder die Suspension verbraucht oder die erforderliche Menge an Aktivkomponente auf dem Träger aufgebracht ist.

Nach einer besonders bevorzugten erfindungsgemäßen Ausführungsform wird die katalytisch aktive Masse des erfindungsgemäßen Katalysators, enthaltend das $TiO_2$ wie hierin definiert, im Fließbett oder Wirbelbett unter Beihilfe geeigneter Bindemittel aufgebracht, so dass ein Schalenkatalysator erzeugt wird. Geeignete Bindemittel umfassen dem Fachmann geläufige organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat sowie Vinylacetat/Ethylen. Besonders bevorzugt wird ein organischer polymerer oder copolymerer Kleber, insbesondere ein Vinylacetat-Copolymer-Kleber, als Bindemittel verwendet. Das verwendete Bindemittel wird in üblichen Mengen der katalytisch aktiven Masse zugegeben, beispielsweise mit etwa 10 bis 20 Gew.-%, bezogen auf den Feststoffgehalt der katalytisch aktiven Masse. Beispielsweise kann auf die EP 744 214 verwiesen werden. Soweit die Aufbringung der katalytisch aktiven Masse bei erhöhten Temperaturen von etwa 150°C erfolgt, ist, wie aus dem Stand der Technik bekannt, eine Aufbringung auf den Träger auch ohne organische Bindemittel möglich. Brauchbare Beschichtungstemperaturen bei Verwendung der vorstehend angegebenen Bindemittel liegen gemäß DE 21 06 796 beispielsweise zwischen etwa 50 und 450°C. Die verwendeten Bindemittel brennen beim Ausheizen des Katalysators bei Inbetriebnahme des gefüllten Reaktors innerhalb kurzer Zeit aus. Die Bindemittel dienen in erster Linie der Verstärkung der Haftung der katalytisch aktiven Masse auf dem Träger und der Verringerung von Abrieb beim Transport und Einfüllen des Katalysators.

Weitere mögliche Verfahren zur Herstellung von Schalenkatalysatoren für die katalytische Gasphasenoxidation von aromatischen Kohlenwasserstoffen zu Carbonsäuren und/oder Carbonsäureanhydriden sind beispielsweise in der WO 98/00778 bzw. EP-A 714 700 beschrieben worden. Danach wird aus einer Lösung und/oder einer Suspension der katalytisch aktiven Metalloxide und/oder deren Vorläuferverbindungen, gegebenenfalls in Anwesenheit von Hilfsmitteln für die Katalysatorherstellung, zunächst ein Pulver hergestellt, das anschließend für die Katalysatorherstellung auf dem Träger, gegebenenfalls nach Konditionierung sowie gegebenenfalls nach Wärmebehandlung zur Erzeugung der katalytisch aktiven Metalloxide schalenförmig aufgebracht und der auf diese Weise beschichtete Träger einer Wärmebehandlung zur Erzeugung der katalytisch aktiven Metalloxide oder einer Behandlung zur Entfernung flüchtiger Bestandteile unterzogen.

Geeignete Bedingungen zur Durchführung eines Verfahrens zur Gasphasenoxidation von Kohlenwasserstoffen, insbesondere zur Herstellung von Phthalsäureanhydrid aus o-Xylol und/oder Naphthalin sind dem Fachmann gleichermaßen aus dem Stand der Technik bekannt. Insbesondere wird auf die zusammenfassende Darstellung in K. Towae, W. Enke, R. Jäckh, N. Bhargana "Phtalic Acid and Derivatives" in Ullmann's Encyclopedia of Industrial Chemistry Vol. A. 20, 1992, 181 verwiesen und diese hiermit durch Bezugnahme aufgenommen. Beispielsweise können für den stationären Betriebszustand der Oxidation die aus der vorstehenden Literaturstelle der WO-A 98/37967 oder der WO 99/61433 bekannten Randbedingungen gewählt werden.

Dazu werden zunächst die Katalysatoren in die Reaktionsrohre des Reaktors, die von außen auf die Reaktionstemperatur, beispielsweise mittels Salzschmelzen thermostatisiert sind, gefüllt. Über die so bereitete Katalysatorschüttung wird das Reaktionsgas bei Temperaturen von im Allgemeinen 300 bis 450°C, vorzugsweise 320 bis 420°C, und besonders bevorzugt von 340 bis 400°C und bei einem Überdruck von im Allgemeinen 0,1 bis 2,5, vorzugsweise von 0,3 bis 1,5 bar mit einer Raumgeschwindigkeit von im Allgemeinen 750 bis 5000 h$^{-1}$ geleitet.

Das dem Katalysator zugeführte Reaktionsgas wird vorzugsweise im Allgemeinen durch Vermischen von einem molekularen Sauerstoff enthaltenden Gas, das außer Sauerstoff noch geeignete Reaktionsmoderatoren und/oder Verdünnungsmittel wie Dampf, Kohlendioxid und/oder Stickstoff enthalten kann, mit dem zu oxidierenden, aromatischen Kohlenwasserstoff erzeugt, wobei das den molekularen Sauerstoff enthaltende Gas im Allgemeinen 1 bis 100, vorzugsweise 2 bis 50 und besonders bevorzugt 10 bis 30 mol-% Sauerstoff, 0 bis 30, vorzugsweise 0 bis 10 mol-% Wasserdampf sowie 0 bis 50, vorzugsweise 0 bis 1 mol-% Kohlendioxid, Rest Stickstoff, enthalten kann. Zur Erzeugung des Reaktionsgases wird das den molekularen Sauerstoff enthaltende Gas im Allgemeinen mit 30 bis 150 g je $Nm^3$ Gas des zu oxidierenden, aromatischen Kohlenwasserstoffs beschickt.

Nach einer besonders bevorzugten erfindungsgemäßen Ausführungsform weist der erfindungsgemäße Katalysator einen Aktivmassegehalt zwischen etwa 7 und 12 Gew.-%, bevorzugt zwischen 8 und 10 Gew.-% auf. Die Aktivmasse (katalytisch aktive Masse) enthält vorzugsweise zwischen 5 bis 15 Gew.-% $V_2O_5$, 0 bis 4 Gew.- $Sb_2O_3$, 0,2 bis 0,75 Gew.-% Cs, 0 bis 3 Gew.-% $Nb_2O_5$ enthält, Neben den vorstehenden Komponenten besteht der Rest der Aktivmasse zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus $TiO_2$. Ein solcher erfindungsgemäßer Katalysator kann beispielsweise vorteilhaft bei einem Zwei- oder Mehrlagen-Katalysator als erste, zur Gaseintrittsseite hin gelegene Katalysatorlage verwendet werden.

Nach einer besonders bevorzugten erfindungsgemäßen Ausführungsform liegt dabei die BET-Oberfläche des Katalysators zwischen 15 und etwa 25 $m^2$/g. Weiterhin wird bevorzugt, dass eine solche erste Katalysatorlage einen Längenanteil von etwa 40 bis 60% an der Gesamtlänge aller vorhandenen Katalysatorlagen (Gesamtlänge des vorhandenen Katalysatorbettes) aufweist.

Nach einer weiteren bevorzugten erfindungsgemäßen Ausführungsform weist der erfindungsgemäße Katalysator einen Aktivmassegehalt von etwa 6 bis 11 Gew.-%, insbesondere 7 bis 9 Gew.-% auf. Die aktive Masse enthält vorzugsweise 5 bis 15 Gew.-% $V_2O_5$, 0 bis 4 Gew.-% $Sb_2O_3$, 0,05 bis 0,3 Gew.-% Cs, 0 bis 2 Gew.-% $Nb_2O_5$ und 0 - 2 Gew.-% Phosphor enthält. Neben den vorstehenden Komponenten besteht der Rest der Aktivmasse zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus $TiO_2$. Ein solcher erfindungsgemäßer Katalysator kann beispielsweise vorteilhaft als zweite Katalysatorlage, d.h. stromab der zur Gaseintrittsseite hin gelegenen ersten Katalysatorlage (vgl. oben) eingesetzt werden. Dabei wird bevorzugt, dass der Katalysator eine BET-Oberfläche zwischen etwa 15 und 25 $m^2$/g aufweist. Weiterhin wird bevorzugt, dass diese zweite Lage einen Längenanteil von etwa 10 bis 30% der Gesamtlänge aller vorhandenen Katalysatorlagen einnimmt .

Nach einer weiteren erfindungsgemäßen Ausführungsform weist der erfindungsgemäße Katalysator einen Aktivmassegehalt zwischen etwa 5 und 10 Gew.-%, insbesondere zwischen 6 und 8 Gew.-% auf. Die Aktivmasse (katalytisch aktive Masse) enthält vorzugsweise 5 bis 15 Gew.-% $V_2O_5$, 0 bis 4 Gew.-% $Sb_2O_3$, 0 bis 0,1 Gew.-% Cs, 0 bis 1 Gew.-% $Nb_2O_5$ und 0 - 2 Gew.-% Phosphor enthält. Neben den vorstehenden Komponenten besteht der Rest der Aktivmasse zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus $TiO_2$. Ein solcher erfindungsgemäßer Katalysator kann beispielsweise vorteilhaft als dritte, (bzw. letzte) stromab der vorstehend beschriebenen zweiten Katalysatorlage, angeordnete Katalysatorlage eingesetzt werden. Bevorzugt wird dabei eine BET-Oberfläche des Katalysators, die etwas höher liegt als diejenige der näher zur Gaseintrittsseite hin gelegenen Schichten, insbesondere im Bereich zwischen etwa 25 bis etwa 45 $m^2$/g. Weiterhin wird bevorzugt, dass eine solche dritte Katalysatorlage einen Längenanteil von etwa 10 bis 50% der Gesamtlänge aller vorhandenen Katalysatorlagen einnimmt.

Es wurde auch überraschend gefunden, dass sich die erfindungsgemäßen Mehrlagen- bzw. Mehrschicht-Katalysatoren, insbesondere mit drei oder mehr Schichten, besonders vorteilhaft einsetzen lassen, wenn die einzelnen Katalysatorlagen in einem bestimmten Längenverhältnis zueinander vorliegen.

So weist nach einer besonders bevorzugten erfindungsgemäßen Ausführungsform die erste, zur Gaseintrittsseite hin gelegene Katalysatorlage einen Längenanteil, bezogen auf die Gesamtlänge des Katalysatorbettes, von mindestens 40%, insbesondere mindestens 45%, besonders bevorzugt mindestens 50% auf. Insbesondere bevorzugt wird, dass der Anteil der ersten Katalysatorlage an der Gesamtlänge des Katalysatorbettes zwischen 40 und 70%, insbesondere zwischen 40 und 55%, besonders bevorzugt zwischen 40 und 52% liegt.

Die zweite Lage nimmt vorzugsweise etwa 10 bis 40%, insbesondere etwa 10 bis 30% der Gesamtlänge des Katalysatorbettes ein. Weiterhin wurde überraschend gefunden, dass ein Verhältnis der Länge der dritten Katalysatorlage zur Länge der zweiten Katalysatorlage zwischen etwa 1 und 2, insbesondere zwischen 1,2 bis 1,7 besonders bevorzugt zwischen 1,3 und 1,6, besonders gute Ergebnisse im Hinblick auf die Wirtschaftlichkeit wie die Effizienz der Rohstoffnutzung und Produktivität des Katalysators liefert.

Es hat sich gezeigt, dass durch die vorstehende Wahl der Längenanteile der einzelnen Katalysatorlagen eine besonders günstige Positionierung des Hot Spots, insbesondere in der ersten Lage, und eine gute Temperaturführung zur Vermeidung zu hoher Hot Spot-Temperaturen auch bei längerer Betriebsdauer des Katalysators ermöglicht wird.

Dadurch wird die Ausbeute, insbesondere bezogen auf die Lebensdauer des Katalysators, verbessert.

Das Temperaturmanagement bei der Gasphasenoxidation von o-Xylol zu Phthalsäureanhydrid ist dem Fachmann aus dem Stand der Technik hinreichend bekannt, wobei beispielsweise auf die DE 100 40 827 A1 verwiesen werden kann.

Weiterhin wird erfindungsgemäß bevorzugt, dass beim Einsatz des erfindungsgemäßen Katalysators in einem Mehrlagen-Katalysatorbett zur Herstellung von Phthalsäureanhydrid der Gehalt an Alkalimetallen in den Katalysatorlagen von der Gaseintrittsseite zur Gasaustrittsseite hin sinkt. Nach einer besonders bevorzugten Ausführungsform ist der Alkaligehalt, bevorzugt der Cs-Gehalt (berechnet als Cs), in der zweiten Katalysatorlage kleiner als in der ersten Katalysatorlage, und in der dritten Katalysatorlage kleiner als in der zweiten Katalysatorlage (und vorzugsweise ggf. auf die dritte Lage folgenden Lagen). Besonders bevorzugt nimmt somit der Cs-Gehalt (berechnet als Cs) im erfindungsgemäßen Katalysator von Lage zu Lage in Gasstromrichtung ab. Nach einer bevorzugten Ausführungsform enthält die dritte (und vorzugsweise auch ggf. nachfolgende Katalysatorlagen) kein Cs. Bevorzugt gilt:

Cs-Gehalt 1. Lage> Cs-Gehalt 2. Lage >... > Cs-Gehalt letzte Lage.

Besonders bevorzugt weist die letzte Katalysatorlage kein Cs auf.

Nach einer besonders bevorzugten Ausführungsform weist nur die letzte Katalysatorlage Phosphor auf. In einer weiteren besonders bevorzugten Ausführungsform ist in der Aktivmasse in der 1. Lage und in der 2. Lage kein Phosphor enthalten. (Mit "kein Phosphor enthalten" ist gemeint, dass bei der Präparation kein P aktiv der Aktivmasse zugegeben wurde).

Auch hat sich überraschend gezeigt, dass in vielen Fällen besonders günstige Drei- oder Mehrlagen-Katalysatoren erhalten werden können, wenn der Aktivmassengehalt von der ersten, zur Gaseintrittsseite hin gelegenen Katalysatorlage zu der zur Gasaustrittsseite hin gelegenen Katalysatorlage abnimmt. Dabei hat sich als vorteilhaft herausgestellt, dass die erste Katalysatorlage einen Aktivmassengehalt zwischen etwa 7 und 12 Gew.-%, insbesondere zwischen etwa 8 und 11 Gew.-%, aufweist, die zweite Katalysatorlage einen Aktivmassengehalt zwischen etwa 6 und 11 Gew.-%, insbesondere zwischen etwa 7 und 10 Gew.-%, aufweist, und die dritte Katalysatorlage einen Aktivmassengehalt zwischen etwa 5 und 10 Gew.-%, insbesondere zwischen etwa 6 und 9 Gew.-%, aufweist.

Die Ausdrücke erste, zweite bzw. dritte Katalysatorlage werden im Zusammenhang mit der vorliegenden Erfindung wie folgt verwendet: als erste Katalysatorlage wird die zur Gaseintrittsseite hin gelegene Katalysatorlage bezeichnet. Zur Gasaustrittsseite hin sind im erfindungsgemäßen Katalysator noch zwei weitere Katalysatorlagen enthalten, die als zweite bzw. dritte Katalysatorlage bezeichnet werden. Die dritte Katalysatorlage liegt dabei näher zur Gasaustrittsseite als die zweite Katalysatorlage.

Nach einer besonders bevorzugten erfindungsgemäßen Ausführungsform weist der erfindungsgemäße Katalysator drei Katalysatorlagen auf. Dann liegt die dritte Katalysatorlage an der Gasaustrittsseite. Die Anwesenheit von zusätzlichen Katalysatorlagen gasstromab der ersten Katalysatorlage ist jedoch nicht ausgeschlossen. Beispielsweise kann nach einer erfindungsgemäßen Ausführungsform der dritten Katalysatorlage wie hierin definiert noch eine vierte Katalysatorlage (mit einem gleichen oder noch geringeren Aktivmassegehalt als die dritte Katalysatorlage) nachfolgen.

Erfindungsgemäß kann nach einer Ausführungsform der Aktivmassegehalt zwischen der ersten und der zweiten Katalysatorlage und/oder zwischen der zweiten und der dritten Katalysatorlage abnehmen. Nach einer besonders bevorzugten erfindungsgemäßen Ausführungsform nimmt der Aktivmassegehalt zwischen der zweiten und der dritten Katalysatorlage ab. Nach einer weiteren bevorzugten erfindungsgemäßen Ausführungsform nimmt dabei die BET-Oberfläche von der ersten, zur Gaseintrittsseite hin gelegenen Katalysatorlage zur dritten, zur Gasaustrittsseite hin gelegenen Katalysatorlage zu. Bevorzugte Bereiche für die BET-Oberfläche sind 15 bis 25 $m^2$/g für die erste Katalysatorlage, 15 bis 25 $m^2$/g für die zweite Katalysatorlage und 25 bis 45 $m^2$/g für die dritte Katalysatorlage.

In vielen Fällen wird erfindungsgemäß bevorzugt, dass die BET-Oberfläche der ersten Katalysatorlage geringer ist als die BET-Oberfläche der dritten Katalysatorlage. Besonders vorteilhafte Katalysatoren werden auch erhalten, wenn die BET-Oberflächen der ersten und der zweiten Katalysatorlage gleich sind, während die BET-Oberfläche der dritten Katalysatorlage demgegenüber größer ist. Die Katalysatoraktivität zur Gaseintrittsseite ist nach einer bevorzugten erfindungsgemäßen Ausführungsform geringer als die Katalysatoraktivität zur Gasaustrittsseite hin.

Dabei wird weiterhin bevorzugt, dass mindestens 0,05 Gew.-% der katalytisch aktiven Masse durch mindestens ein Alkalimetall, berechnet als Alkalimetall(e), gebildet wird. Besonders bevorzugt wird als Alkalimetall Cäsium verwendet.

Zudem wird nach den Ergebnissen der Erfinder nach einer Ausführungsform bevorzugt, dass der erfindungsgemäße Katalysator insgesamt Niob in einer Menge von 0,01 bis 2 Gew.-%, insbesondere 0,5 bis 1 Gew.-% der katalytisch aktiven Masse enthält.

Die erfindungsgemäßen Katalysatoren werden in üblicher Weise vor dem Einsatz temperaturbehandelt bzw. calciniert (konditioniert). Dabei hat sich als vorteilhaft herausgestellt, wenn der Katalysator mindestens 24 Stunden bei mindestens 390°C, insbesondere zwischen 24 und 72 Stunden bei mindestens 400°C, in einem $O_2$-haltigen Gas, insbesondere in Luft, calciniert wird. Die Temperaturen sollten vorzugsweise etwa 500°C, insbesondere etwa 470°C, nicht übersteigen. Grundsätzlich sind jedoch auch andere Calcinierungsbedingungen, die dem Fachmann als geeignet erscheinen, nicht ausgeschlossen.

Nach einem weiteren Aspekt betrifft die vorliegende Erfindung ein Verfahren zur Herstellung eines Katalysators gemäß einem der vorstehenden Ansprüche, umfassend die folgenden Schritte:

a. Bereitstellen einer katalytisch aktiven Masse wie hierin definiert, enthaltend das vorstehend näher charakterisierte $TiO_2$;

b. Bereitstellen eines inerten Trägers, insbesondere eines inerten Trägerformkörpers;

c. Aufbringen der katalytisch aktiven Masse auf den inerten Träger, insbesondere in einer Wirbelschicht oder einem Fließbett.

Es wird dann vorzugsweise getrocknet und calciniert. Nach einem weiteren Aspekt betrifft die vorliegende Erfindung auch die Verwendung eines Titandioxids wie vorstehend definiert zur Herstellung eines Katalysators, insbesondere zur Gasphasenoxidation von Kohlenwasserstoffen, vorzugsweise zur Gasphasenoxidation von o-Xylol und/oder Naphthalin zu Phthalsäureanhydrid.

## METHODEN

Zur Bestimmung der Parameter der erfindungsgemäßen Katalysatoren werden die nachstehenden Methoden eingesetzt:

1. BET-Oberfläche:

Die Bestimmung erfolgt nach der BET-Methode gemäß DIN 66131; eine Veröffentlichung der BET-Methode findet sich auch in J. Am. Chem. Soc. 60, 309 (1938).

2. Porenradienverteilung:

Die Bestimmung der Porenradienverteilung und des Porenvolumens des eingesetzten $TiO_2$ erfolgte mittels Quecksilberporosimetrie gemäß DIN 66133; maximaler Druck: 2.000 bar, Porosimeter 4000 (Firma Porotec, DE), nach Angaben des Herstellers.

3. Primärkristallitgrößen:

Die Bestimmung der Primärkristallitgrößen (Primärpartikelgrö-ßen) erfolgte mittels Pulver-Röntgendiffraktometrie. Die Analyse wurde mit einem Gerät der Firma Bruker, DE, durchgeführt: Typ BRUKER AXS - D4 Endeavor. Die erhaltenen Röntgendiffraktogramme wurden mit dem Softwarepaket "DiffracPlus D4 Measurement" gemäß den Angaben des Herstellers aufgezeichnet und die Halbwertsbreite des 100% Reflexes wurde mit der Software "DiffracPlus Evaluation" nach der Debye-Scherrer Formel gemäß den Angaben des Herstellers ausgewertet, um die Primärkristallitgröße zu bestimmen.

4. Teilchengrößen:

Die Bestimmung der Teilchengrößen erfolgte nach der Laserbeugungsmethode mit einem Fritsch Particle Sizer Analysette 22 Economy (Fa. Fritsch, DE) nach den Angaben des Herstellers, auch bezüglich der Probenvorbehandlung: die Probe wird in deionisiertem Wasser ohne Zusatz von Hilfsmitteln homogenisiert und 5 Minuten mit Ultraschall behandelt.

5. Bestimmung von Verunreinigungen des $TiO_2$:

Die Bestimmung der chemischen Verunreinigungen des $TiO_2$, insbesondere der Gehalte an S, P und Nb erfolgt nach DIN ISO 9964-3. So können die Gehalte mittels ICP-AES (Inductively Coupled Plasma Atomic Emission Spectroscopy) bestimmt und ggf. im Falle von Alkali zum Gesamtalkaligehalt des $TiO_2$ aufaddiert werden.

6. Schüttdichte:

Die Schüttdichte wurde anhand des zur Herstellung des Katalysators eingesetzten $TiO_2$ (bei 150°C im Vakuum getrocknet, uncalciniert) bestimmt. Die erhaltenen Werte aus drei Bestimmungen wurden gemittelt.

Die Schüttdichte wurde bestimmt, indem 100 g des TiO$_2$-Materials in eine 1.000 ml-Dose eingefüllt und ca. 30 Sekunden geschüttelt wurden.

Ein Messzylinder (Fassungsvermögen genau 100ml) wird leer auf 10 mg gewogen. Darauf wird der Pulvertrichter mit Stativ und Klemme über die Öffnung des Zylinders befestigt. Nach Ingangsetzung der Stoppuhr wird der Messzylinder innerhalb von 15 Sekunden mit dem TiO$_2$-Material gefüllt. Mit dem Spatel wird laufend Füllgut nachgeschüttet, so dass der Messzylinder immer leicht überstehend gefüllt ist. Nach 2 Minuten wird mit dem Spatel der Überstand abgestreift, wobei darauf zu achten ist, dass keine Presskräfte das Material im Zylinder verdichten. Der gefüllte Messzylinder wird abgepinselt und gewogen.

Die Schüttdichte wird in g/l angegeben.

Die Bestimmung der BET-Oberfläche, der Porenradienverteilung bzw. des Porenvolumens sowie der Primärkristallitgrößen und der Teilchengrößenverteilung erfolgte bezüglich des Titandioxids jeweils an dem bei 150°C im Vakuum getrockneten, uncalcinierten Material.

Auch die Angaben in der vorliegenden Beschreibung bezüglich der BET-Oberflächen der Katalysatoren bzw. Katalysatorlagen beziehen sich auf die BET-Oberflächen des jeweils eingesetzten TiO$_2$-Materials (getrocknet in Vakuum bei 150°C, uncalciniert, vgl. oben).

In der Regel wird die BET-Oberfläche des Katalysators durch die BET-Oberfläche des eingesetzten TiO$_2$ bestimmt, wobei durch den Zusatz weiterer katalytisch aktiver Komponenten die BET-Oberfläche in gewissem Umfang verändert wird. Dies ist dem Fachmann geläufig.

Der Aktivmasseanteil (Anteil der katalytisch aktiven Masse, ohne Bindemittel) bezieht sich jeweils auf den Anteil (in Gew.-%) der katalytisch aktiven Masse an dem Gesamtgewicht des Katalysators einschließlich Träger in der jeweiligen Katalysatorlage, gemessen nach Konditionierung über 4h bei 400°C.

Die Erfindung wird nun anhand der nachstehenden, nicht beschränkenden Beispiele näher erläutert:

## BEISPIELE

**Beispiel 1:** Herstellung von Katalysator A (Vergleich)

Zur Herstellung des Katalysators A mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 1,1 g Cäsiumsulfat, 1,5 g Ammoniumdihydrogenphosphat, 178,6 g Titandioxid mit einer BET-Oberfläche von 19 m$^2$/g (Firma Nano Co. Ltd., 1108-1 Bongkok Sabong, Jinju, Kyoungnam 660-882 Korea, Handelsname NT22) und folgenden chemischen Verunreinigungen:

- S : 1450 ppm
- P : 760 ppm
- Nb : 1180 ppm
- Summe (Alkali) : 280 ppm

120,5 g Bindemittel aus einer 50%-igen Dispersion von Wasser und Vinylacetat/Ethylencopolymer (Vinnapas® EP 65 W, Fa. Wacker) und 1000 g Wasser bei einer Temperatur von 70 °C beschichtet. Die Aktivmasse wurde in Form dünner Schichten aufgetragen.

**Beispiel 2:** Herstellung von Katalysator B (erfindungsgemäß)

Vor der eigentlichen Herstellung des Katalysators B wurden 200 g des TiO$_2$ gemäß Beispiel 1 in jeweils mehreren Wasch- und Filtrierschritten zuerst mit 1 molarer Salpetersäure, bidestilliertem Wasser, 1 molarem Ammoniakwasser und abschließend erneut mit bidestilliertem Wasser jeweils für 12 h unter Rühren gewaschen und abfiltriert. Abschließend wurde die Probe getrocknet. Das gewaschene TiO$_2$-Material wies folgende chemische Verunreinigungen auf:

- S : 850 ppm
- P: 450 ppm
- Nb : 1170 ppm
- Summe(Alkali): 250 ppm

Zur Herstellung des Katalysators B mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden dann in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 1,1 g Cäsiumsulfat, 1,5 g Ammoniumdihydrogenphosphat, 178,6 g des wie vorstehend beschrieben gewaschenen Titandioxids (BET-Oberfläche 19 $m^2$/g), 120,5 g Bindemittel aus einer 50%-igen Dispersion von Wasser und Vinylacetat/Ethylencopolymer (Vinnapas® EP 65 W, Fa. Wacker) und 1000 g Wasser bei einer Temperatur von 70 °C beschichtet. Die Aktivmasse wurde in Form dünner Schichten aufgetragen.

**Beispiel 3:** Herstellung von Katalysator C (erfindungsgemäß)

Vor der eigentlichen Herstellung des Katalysators C wurden 200 g des $TiO_2$ eines bereits gemäß Beispiel 2 gewaschenen $TiO_2$ in jeweils mehreren Wasch- und Filtrierschritten zuerst mit 1 molarer Salpetersäure, bidestilliertem Wasser, 1 molarem Ammoniakwasser und abschließend erneut mit bidestilliertem Wasser jeweils für 12 h unter Rühren gewaschen und abfiltriert. Abschließend wurde die Probe getrocknet. Das gewaschene $TiO_2$-Material wies folgende chemische Verunreinigungen auf:

- S: 290 ppm
- P: 260 ppm
- Nb: 1150 ppm
- Summe (Alkali) : 230 ppm

Zur Herstellung des Katalysators C mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden dann in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 1,1 g Cäsiumsulfat, 1,5 g Ammoniumdihydrogenphosphat, 178,6 g des wie vorstehend beschrieben gewaschenen Titandioxids (BET-Oberfläche 19 $m^2$/g), 120,5 g Bindemittel aus einer 50%-igen Dispersion von Wasser und Vinylacetat/Ethylencopolymer (Vinnapas® EP 65 W, Fa. Wacker) und 1000 g Wasser bei einer Temperatur von 70 °C beschichtet. Die Aktivmasse wurde in Form dünner Schichten aufgetragen.

**Beispiel 4:** Herstellung von Katalysator D (erfindungsgemäß)

Vor der eigentlichen Herstellung des Katalysators D wurden 200 g eines bereits gemäß Beispiel 3 gewaschenen $TiO_2$ in jeweils mehreren Wasch- und Filtrierschritten zuerst mit 1 molarer Salpetersäure, bidestilliertem Wasser, 1 molarem Ammoniakwasser und abschließend erneut mit bidestilliertem Wasser jeweils für 12 h unter Rühren gewaschen und abfiltriert. Abschließend wurde die Probe getrocknet. Das gewaschene $TiO_2$-Material wies folgende chemische Verunreinigungen auf:

- S : 140 ppm
- P: 200 ppm
- Nb : 1160 ppm
- Summe (Alkali) : 230 ppm

Zur Herstellung des Katalysators D mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden dann in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 1,1 g Cäsiumsulfat, 1,5 g Ammoniumdihydrogenphosphat, 178,6 g des wie vorstehend beschrieben gewaschenen Titandioxids (BET-Oberfläche 19 $m^2$/g), 120,5 g Bindemittel aus einer 50%-igen Dispersion von Wasser und Vinylacetat/Ethylencopolymer (Vinnapas® EP 65 W, Fa. Wacker) und 1000 g Wasser bei einer Temperatur von 70 °C beschichtet. Die Aktivmasse wurde in Form dünner Schichten aufgetragen.

**Beispiel 5:** Ermittlung der katalytischen Leistungsdaten der Katalysatoren A, B, C und D

In ein 120 cm langes Reaktionsrohr mit einem Innendurchmesser von 24,8 mm werden 40 g Katalysator A, verdünnt mit 200 g Steatitringen der Abmessungen 8 x 6 x 5 mm zur Vermeidung von Hotspots, auf einer Länge von 80 cm eingefüllt. Das Reaktionsrohr befindet sich in einer flüssigen Salzschmelze die auf Temperaturen bis 450 °C aufgeheizt werden kann. In der Katalysatorschüttung befindet sich ein 3 mm Schutzrohr mit eingebautem Thermoelement über das die Katalysatortemperatur über die komplette Katalysatorkombination angezeigt werden kann. Zur Ermittlung der katalytischen Leistungsdaten werden über den Katalysator A 60 g/Nm³ o-Xylol (Reinheit 99,9%) bei maximal 400 N1 Luft/h geleitet. Anschließend wird die Salzbadtemperatur dahingehend angepasst, dass der o-Xylolumsatz zwischen 55 und 65 % liegt. Die Ergebnisse des Testlaufs sind in der Tabelle 1 aufgeführt.

Entsprechend wird mit den Katalysatoren B, C und D in parallelen Testläufen verfahren. Die Ergebnisse der Testläufe sind in der Tabelle 1 aufgeführt.

Tabelle 1

| Beispiel | Umsatz [%] | Salzbadtemperatur [°C] | $C_8$-Selektivität [mol.%] | Selektivität PSA [mol.-%] | Selektivität $CO_x$ [mol.-%] | Selektivität MSA[mol.-%] |
|---|---|---|---|---|---|---|
| Kat. A (Bsp. 1) | 59,4 | 380 | 82,8 | 70,9 | 12,5 | 3,0 |
| Kat. B (Bsp. 2) | 63,2 | 380 | 84,5 | 74,7 | 11,3 | 2,1 |
| Kat. C (Bsp. 3) | 62,8 | 380 | 86,0 | 76,1 | 10,5 | 1,4 |
| Kat. D (Bsp. 4) | 64,4 | 380 | 86,6 | 76,6 | 10,2 | 1,5 |
| $C_8$-Selektivität: Selektivität bzgl. aller Wertprodukte mit 8 Kohlenstoffatomen (Phthalsäureanhydrid, Phthalid, o-Tolylaldehyd, o-Tolylsäure) $CO_x$: Summe aus Kohlenmonoxid und -dioxid im Abgasstrom PSA: Phthalsäureanhydrid; MSA: Maleinsäureanhydrid; Kat.: Katalysator | | | | | | |

Aus Tabelle 1 geht klar hervor, dass sowohl der Umsatz, als auch die $C_8$- und die PSA-Selektivität bei den erfindungemäßen Katalysatoren (Katalysatoren B, C und D) deutlich höher liegen, als bei dem Vergleichsmaterial (Katalysator A). Weiterhin fällt die Bildung von MSA als Nebenprodukt bei den erfindungemäßen Katalysatoren deutlich geringer aus als bei dem Vergleichsmaterial. Es zeigt sich auch, dass die verbesserten Eigenschaften der Katalysatoren nicht auf einen unterschiedlichen Alkaligehalt der verwendeten Materialien zurückzuführen sind, da sich die Katalysatoren A bis D in dieser Hinsicht nicht erheblich unterscheiden.

**Beispiel 6:** Herstellung von Katalysator E (Vergleich)

Zur Herstellung des Katalysators E mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 1, 1 g Cäsiumsulfat, 1,5 g Ammoniumdihydrogenphosphat, 178,6 g eines kommerziell erhältlichen Titandioxids mit einer BET-Oberfläche von 20 m²/g und folgender chemischer Verunreinigungen

- S : 2230 ppm
- P : 880 ppm
- Nb : 1530 ppm

120,5 g Bindemittel (siehe Beispiel 1) und 1000 g Wasser bei einer Temperatur von 70 °C beschichtet. Die Aktivmasse wurde in Form dünner Schichten aufgetragen.

**Beispiel 7:** Herstellung von Katalysator F (Erfindungsgemäß)

Zur Herstellung des Katalysators F mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 1,1 g Cäsiumsulfat, 1,5 g Ammoniumdihydrogenphosphat, 178,6 g Titandioxid mit einer BET-Oberfläche von 19 m²/g (durch Waschschritte gemäß Beispiel 2 aus einem anderen kommerziell erhältlichen TiO₂ erhalten) und folgenden chemischen Verunreinigungen

- S: 120 ppm
- P: 220 ppm
- Nb: 1160 ppm

120,5 g Bindemittel (siehe Beispiel 1) und 1000 g Wasser bei einer Temperatur von 70 °C beschichtet. Die Aktivmasse wurde in Form dünner Schichten aufgetragen.

**Beispiel 8:** Herstellung von Katalysator G (Erfindungsgemäß)

Zur Herstellung des Katalysators G mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 1,1 g Cäsiumsulfat, 1,5 g Ammoniumdihydrogenphosphat, 178,6 g Titandioxid mit einer BET-Oberfläche von 20 m$^2$/g (durch Waschschritte gemäß Beispiel 2 aus einem anderen kommerziell erhältlichen $TiO_2$ erhalten) und folgenden chemischen Verunreinigungen

- S : 250 ppm
- P: 240 ppm
- Nb : 1350 ppm

120,5 g Bindemittel (siehe Beispiel 1) und 1000 g Wasser bei einer Temperatur von 70 °C beschichtet. Die Aktivmasse wurde in Form dünner Schichten aufgetragen.

**Beispiel 9:** Herstellung von Katalysator H (Erfindungsgemäß)

Zur Herstellung des Katalysators H mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 1,1 g Cäsiumsulfat, 1,5 g Ammoniumdihydrogenphosphat, 178,6 g Titandioxid mit einer BET-Oberfläche von 19 m$^2$/g (durch Waschschritte gemäß Beispiel 2 aus einem anderen kommerziell erhältlichen $TiO_2$ erhalten) und folgenden chemischen Verunreinigungen:

- S : 480 ppm
- P : 620 ppm
- Nb : 1800 ppm

120,5 g Bindemittel (siehe Beispiel 1) und 1000 g Wasser bei einer Temperatur von 70 °C beschichtet. Die Aktivmasse wurde in Form dünner Schichten aufgetragen.

**Beispiel 10**: Ermittlung der katalytischen Leistungsdaten der Katalysatoren E bis H

In ein 120 cm langes Reaktionsrohr mit einem Innendurchmesser von 24,8 mm werden 40 g Katalysator E, verdünnt mit 200 g Steatitringen der Abmessungen 8 x 6 x 5 mm zur Vermeidung von Hotspots, auf einer Länge von 80 cm eingefüllt. Das Reaktionsrohr befindet sich in einer flüssigen Salzschmelze die auf Temperaturen bis 450 °C aufgeheizt werden kann. In der Katalysatorschüttung befindet sich ein 3 mm Schutzrohr mit eingebautem Thermoelement über das die Katalysatortemperatur über die komplette Katalysatorkombination angezeigt werden kann. Zur Ermittlung der katalytischen Leistungsdaten werden über den Katalysator A 60 g/Nm$^3$ o-Xylol (Reinheit 99,9%) bei maximal 400 N1 Luft/h geleitet. Anschließend wird die Salzbadtemperatur dahingehend angepasst, dass der o-Xylolumsatz zwischen 55 und 65 % liegt. Die Ergebnisse des Testlaufs sind in der Tabelle 1 aufgeführt.
Entsprechend wird mit den Katalysatoren F, G und H in parallelen Testläufen verfahren. Die Ergebnisse der Testläufe sind in der Tabelle 2 aufgeführt.

Tabelle 2

| Beispiel | Umsatz (%) | Salzbadtemperatur [°C] | $C_8$-Selektivität [mol.-%] | Selektivität PSA [mol.-%] | Selektivität $CO_x$ [mol.-%] | Selektivität MSA [mol.-%] |
|---|---|---|---|---|---|---|
| Katalysator E (Bsp. 6) | 57,4 | 376 | 83,2 | 72,1 | 12,5 | 3,3 |
| Katalysator F (Bsp. 7) | 64,8 | 376 | 87,1 | 78,3 | 10,3 | 8 1,8 |
| Katalysator G (Bsp. 8) | 63,4 | 376 | 86,9 | 77,2 | 10,5 | 2,2 |
| Katalysator H (Bsp. 9) | 60,6 | 376 | 85,2 | 75,6 | 11,2 | 2,8 |

$C_8$-Selektivität: Selektivität bzgl. aller Wertprodukte mit 8 Kohlenstoffatomen (Phthalsäureanhydrid, Phthalid, o-Tolylaldehyd, o-Tolylsäure)

$CO_x$: Summe aus Kohlenmonoxid und -dioxid im Abgasstrom PSA: Phthalsäureanhydrid MSA: Maleinsäureanhydrid

**Beispiel 11:** Herstellung eines erfindungsgemäßen Dreischicht-Katalysators

Ein erfindungsgemäßer Dreischicht-Katalysator kann beispielsweise wie folgt erhalten werden:

Zur Herstellung eines Katalysators J mit einem Aktivmassenanteil von 9 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid (wie in Beispiel 3) wurden in einem sogenannten Wirbelbett-Coater 2200 g Steatitkörper in Form von Hohlzylindern der Größe 8 x 6 x 5 mm mit einer Suspension aus 17,2 g Vanadiumpentoxid, 7,3 g Antimontrioxid, 1,25 g Cäsiumsulfat, 1,72 g Ammoniumdihydrogenphosphat, 203,2 g Titandioxid mit einer BET-Oberfläche von 19 m$^2$/g, 120 g Bindemittel aus einer 50%-igen Dispersion von Wasser und Vinylacetat/Ethylencopolymer (Vinnapas® EP 65 W, Fa. Wacker) und 1000 g Wasser bei einer Temperatur von 70°C beschichtet. Die aktive Masse wurde in Form dünner Schichten aufgetragen.

Zur Herstellung eines Katalysators K mit einem Aktivmassenanteil von 8 Gew.-% und der Zusammensetzung von 7,5 Gew.-% Vanadiumpentoxid, 3,2 Gew.-% Antimontrioxid, 0,20 Gew.-% Cäsium (berechnet als Cäsium), 0,2 Gew.-% Phosphor (berechnet als Phosphor) und Rest Titandioxid (wie in Beispiel 3) wurden in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 0,5 g Cäsiumsulfat, 1,5 g Ammoniumdihydrogenphosphat, 179 g Titandioxid mit einer BET-Oberfläche von 19 m$^2$/g, 120 g Bindemittel aus einer 50%-igen Dispersion von Wasser und Vinylacetat/Ethylencopolymer (Vinna-pas® EP 65 W, Fa. Wacker) und 1000 g Wasser bei einer Temperatur von 70°C beschichtet. Die aktive Masse wurde in Form dünner Schichten aufgetragen.

Zur Herstellung eines Katalysators L mit einem Aktivmassenanteil von 8 Gew.-% und der Zusammensetzung von 11 Gew.-% Vanadiumpentoxid, 0,35 Gew.-% Phosphor (berechnet als Phosphor) und Rest Titandioxid (wie in Beispiel 3) wurden in einem sogenannten Wirbelbett-Coater 2200 g Steatitkörper in Form von Hohlzylindern der Größe 8 x 6 x 5 mm mit einer Suspension aus 22,2 g Vanadiumpentoxid, 2,6 g Ammoniumdihydrogenphosphat, 178,5 g Titandioxid mit einer BET-Oberfläche von 19 m$^2$/g, 120 g Bindemittel aus einer 50%-igen Dispersion von Wasser und Vinylacetat/Ethylencopolymer (Vinnapas® EP 65 W, Fa. Wacker) und 1000 g Wasser bei einer Temperatur von 70°C beschichtet. Die aktive Masse wurde in Form dünner Schichten aufgetragen.

Die Abfolge der Katalysatorlagen: 140 cm des Katalysators J, 60 cm des Katalysators K, 90 cm des Katalysators L.

**Beispiel 12:** Katalytische Leistungsdaten des erfindungsgemäßen Dreischicht-Katalysators

In ein 450 cm langes Reaktionsrohr werden hintereinander 90 cm des Katalysators L, 60 cm des Katalysators K und 140 cm des Katalysators J gefüllt. Das Reaktionsrohr befindet sich in einer flüssigen Salzschmelze, die auf Temperaturen bis 450°C aufgeheizt werden kann. In der Katalysatorschüttung befindet sich ein 3 mm Schutzrohr mit eingebauten Thermoelement, über das die Katalysatortemperatur über die komplette Katalysatorkombination angezeigt werden kann. Zur Ermittlung der katalytischen Leistungsdaten werden über diese Katalysatorkombination in der Reihenfolge DEF von 0 bis maximal 70 g/Nm$^3$ o-Xylol (Reinheit 99,9%) bei 3,6 Nm$^3$ Luft/h geleitet und das Reaktionsgas nach Reaktionsrohraustritt durch einen Kondensator geleitet, indem sich alle organischen Bestandteile des Reaktionsgases, bis auf das Kohlenmonoxid und Kohlendioxid, abscheiden. Das abgeschiedene Rohprodukt wird mittels überhitzten Dampf abgeschmolzen, aufgefangen und anschließend verwogen.

Die Rohausbeute wird wie folgt bestimmt.

$$\textbf{\textit{Max. Roh-PSA-Ausbeute [Gew.-\%]}}$$

$$=$$

$$\textbf{\textit{Ausgewogene Menge Roh-PSA [g] x 100 / Zulauf o-Xylol [g] x Rein-heit o-Xylol [\%/100]}}$$

Die Ergebnisse des Testlaufs sind in der Tabelle 2 aufgeführt.

Tabelle 3

| Beispiel | Maximal Beladung | Roh-PSA-Ausbeute | PSA-Qualität (Phthalidwert im Reaktionsgas) | Hotspottemperatur und - lage |
|---|---|---|---|---|
| Beispiel 12: Katalysatorkombination J (140 cm) K (60 cm) L (90 cm) | 65 g/Nm$^3$ | 114,4Gew.-% | < 500 ppm | 438 °C 65 cm (1. Lage) |

Wie aus Tabelle 3 ersichtlich, zeigt der erfindungsgemäße Katalysator gemäß Beispiel 12 eine sehr gute PSA-Ausbeute und PSA-Qualität. Der Hot Spot ist vorteilhaft in der ersten Katalysatorlage positioniert.

**Patentansprüche**

1. Katalysator, insbesondere zur Gasphasenoxidation von o-Xylol und/oder Naphthalin, mit einer katalytisch aktiven Masse enthaltend TiO$_2$, wobei das TiO$_2$ einen Gehalt an Schwefel, berechnet als elementarer Schwefel, von weniger als etwa 1000 ppm und eine BET-Oberfläche von mindestens 5 m$^2$/g aufweist.

2. Katalysator nach Anspruch 1, **dadurch gekennzeichnet, dass** der Gehalt des TiO$_2$ an Schwefel, berechnet als elementarer Schwefel, weniger als etwa 750 ppm, bevorzugt weniger als etwa 500 ppm, besonders bevorzugt weniger als etwa 300 ppm, weiter bevorzugt weniger als etwa 200 ppm beträgt.

3. Katalysator nach einem der vorstehenden Ansprüche, **dadurch gekennzeichnet, dass** der Gehalt des TiO$_2$ an Phosphor, berechnet als elementarer Phosphor, weniger als etwa 800 ppm, bevorzugt weniger als etwa 700 ppm, insbesondere weniger als etwa 500 ppm, insbesondere weniger als etwa 300 ppm auf, weiter bevorzugt weniger als etwa 280 ppm beträgt.

4. Katalysator nach einem der vorstehenden Ansprüche, **dadurch gekennzeichnet, dass** er einen inerten Träger und mindestens eine darauf aufgebrachten Schicht mit der aktiven Masse aufweist.

5. Katalysator nach einem der vorstehenden Ansprüche, **dadurch gekennzeichnet, dass** der Gehalt des TiO$_2$ an Niob, berechnet als Nb, mehr als etwa 500 ppm, insbesondere mehr als etwa 1000 ppm beträgt.

6. Katalysator nach nach einem der vorstehenden Ansprüche, **dadurch gekennzeichnet, dass** die BET-Oberfläche des TiO$_2$ zwischen etwa 15 und 60 m$^2$/g, insbesondere etwa 15 und 45 m$^2$/g, besonders bevorzugt 15 und 35 m$^2$/g liegt.

7. Katalysator nach einem der vorstehenden Ansprüche, **dadurch gekennzeichnet, dass** zumindest ein Teil des eingesetzten $TiO_2$ folgende Eigenschaften aufweist: (a) die BET-Oberfläche beträgt mehr als 15 $m^2$/g, (b) mindestens 25% des gesamten Porenvolumens werden durch Poren mit einem Radius zwischen 60 und 400 nm gebildet und (c) die Primärkristallitgröße liegt bei mehr als 210 Angström.

8. Katalysator nach einem der vorstehenden Ansprüche, **dadurch gekennzeichnet, dass** die Schüttdichte bei weniger als 1,0 g/ml, vorzugsweise weniger als 0,8 g/ml, insbesondere weniger als 0,6 g/ml liegt.

9. Katalysator nach einem der vorstehenden Ansprüche, **dadurch gekennzeichnet, dass** die katalytisch aktive Masse im Fließ- bzw. Wirbelbett aufgebracht wird.

10. Katalysator nach einem der vorstehenden Ansprüche, **dadurch gekennzeichnet, dass** der $D_{90}$-Wert des einge-setzten $TiO_2$ zwischen etwa 0,5 und 20 $\mu$m, insbesondere zwischen etwa 1 und 10 $\mu$m, besonders bevorzugt zwischen etwa 2 und 5 $\mu$m liegt.

11. Katalysator nach einem der vorstehenden Ansprüche, **dadurch gekennzeichnet, dass** 4 Gew.-% oder mehr der katalytisch aktiven Masse, insbesondere zwischen etwa 6 und 15 Gew.-% an Vanadium, berechnet als Vanadiumpentoxid, vorliegen.

12. Katalysator nach einem der vorstehenden Ansprüche, **dadurch gekennzeichnet, dass** mindestens 0,05 Gew.-% der katalytisch aktiven Masse an mindestens einem Alkalimetall, berechnet als Alkalimetall, vorliegen.

13. Katalysator nach einem der vorstehenden Ansprüche, **dadurch gekennzeichnet, dass** als Kleber für die katalytisch aktive Masse ein organisches Polymer oder Copolymer, insbesondere ein Vinylacetatcopolymer, verwendet wird.

14. Katalysator nach einem der vorstehenden Ansprüche, **dadurch gekennzeichnet, dass** der Katalysator mindestens 24 Stunden bei > 390°C, bevorzugt zwischen 24 und 72 Stunden bei ≥ 400°C, in einem $O_2$-haltigen Gas, insbesondere in Luft, calciniert bzw. konditioniert wird.

15. Katalysator nach einem der vorstehenden Ansprüche, **dadurch gekennzeichnet, dass** Niob in einer Menge von 0,1 bis 2 Gew.-%, insbesondere 0,5 bis 1 Gew.-% der katalytisch aktiven Masse vorhanden ist.

16. Katalysator nach einem der vorstehenden Ansprüche, **dadurch gekennzeichnet, dass** nur eine $TiO_2$-Quelle ver-wendet wird.

17. Katalysator nach einem der vorstehenden Ansprüche, **dadurch gekennzeichnet, dass** Katalysator nach einem der vorstehenden Ansprüche, **dadurch gekennzeichnet, dass** es sich um einen drei oder mehr Lagen umfassenden Katalysator handelt und Phosphor in der dritten bzw. letzten Lage mit 0,05 bis 0,5 Gew.-% vorliegt.

18. Katalysator nach einem der vorstehenden Ansprüche, enthaltend eine erste, zur Gaseintrittsseite hin gelegene Katalysatorlage, eine zweite, näher zur Gasaustrittsseite hin gelegene Katalysatorlage und eine dritte, noch näher zur oder an der Gasaustrittsseite hin gelegene Katalysatorlage, wobei die Katalysatorlagen unterschiedlich zusammengesetzt sind und jeweils eine Aktivmasse enthaltend $TiO_2$ aufweisen, und wobei der Aktivmassegehalt von der ersten zur dritten Katalysatorlage abnimmt, mit der Maßgabe, dass

a) die erste Katalysatorlage einen Aktivmassegehalt zwischen etwa 7 und 12 Gew.-% aufweist,
b) die zweite Katalysatorlage einen Aktivmassegehalt im Bereich zwischen 6 und 11 Gew.-% aufweist, wobei der Aktivmassegehalt der zweiten Katalysatorlage kleiner oder gleich dem Aktivmassegehalt der ersten Katalysatorlage ist, und
c) die dritte Katalysatorlage einen Aktivmassegehalt im Bereich zwischen 5 und 10 Gew.-% aufweist, wobei der Aktivmassegehalt der dritten Katalysatorlage kleiner oder gleich dem Aktivmassegehalt der zweiten Katalysatorlage ist.

19. Katalysator nach einem der vorstehenden Ansprüche, enthaltend eine erste, zur Gaseintrittsseite hin gelegene Katalysatorlage, eine zweite, näher zur Gasaustrittsseite hin gelegene Katalysatorlage, eine dritte noch näher zur Gasaustrittsseite hin gelegene Katalysatorlage und eine vierte, noch näher zur oder an der Gasaustrittsseite hin gelegene Katalysatorlage, wobei die Katalysatorlagen unterschiedlich zusammengesetzt sind und jeweils eine Ak-tivmasse enthaltend $TiO_2$ aufweisen, und wobei der Aktivmassegehalt von der ersten zur vierten Katalysatorlage abnimmt, mit der Maßgabe, dass

> a) die erste Katalysatorlage einen Aktivmassegehalt zwischen etwa 7 und 12 Gew.-% aufweist,
> b) die zweite Katalysatorlage einen Aktivmassegehalt im Bereich zwischen 6 und 11 Gew.-% aufweist, wobei der Aktivmassegehalt der zweiten Katalysatorlage kleiner oder gleich dem Aktivmassegehalt der ersten Katalysatorlage ist, und
> c) die dritte Katalysatorlage einen Aktivmassegehalt im Bereich zwischen 5 und 10 Gew.-% aufweist, wobei der Aktivmassegehalt der dritten Katalysatorlage kleiner oder gleich dem Aktivmassegehalt der zweiten Katalysatorlage ist;
> d) die vierte Katalysatorlage einen Aktivmassegehalt im Bereich zwischen 4 und 9 Gew.-% aufweist, wobei der Aktivmassegehalt der vierten Katalysatorlage kleiner oder gleich dem Aktivmassegehalt der dritten Katalysatorlage ist.

**20.** Katalysator nach einem der vorstehenden Ansprüche, **dadurch gekennzeichnet, dass** die Katalysatoraktivität der Katalysatorlage zur Gaseintrittsseite hin geringer ist als die Katalysatoraktivität der Katalysatorlage zur Gasaustrittsseite hin.

**21.** Katalysator nach einem der vorstehenden Ansprüche, **dadurch gekennzeichnet, dass** die BET-Oberfläche der ersten Katalysatorlage geringer ist als die BET-Oberfläche der letzten Katalysatorlage.

**22.** Katalysator nach einem der vorstehenden Ansprüche, **dadurch gekennzeichnet, dass** der Anteil der ersten Katalysatorlage an der Gesamtlänge des Katalysatorbettes zwischen 40 und 70%, insbesondere zwischen 40 und 55%, besonders bevorzugt zwischen 40 und 52%, liegt.

**23.** Katalysator nach einem der vorstehenden Ansprüche, **dadurch gekennzeichnet, dass** der Anteil der zweiten Katalysatorlage an der Gesamtlänge des Katalysatorbettes zwischen etwa 10 und 40%, insbesondere zwischen etwa 10 und 30%, liegt.

**24.** Verfahren zur Herstellung eines Katalysators gemäß einem der vorstehenden Ansprüche, umfassend die folgenden Schritte:

> a. Bereitstellen einer aktiven Masse, enthaltend $TiO_2$ wie in einem der vorstehenden Ansprüche definiert,
> b. Bereitstellen eines inerten Trägers, insbesondere eines inerten Trägerformkörpers,
> c. Aufbringen der katalytisch aktiven Masse auf den inerten Träger insbesondere in einer Wirbelschicht oder einem Fließbett.

**25.** Verwendung eines Titandioxids mit einem Gehalt an Schwefel, berechnet als elementarer Schwefel, von weniger als etwa 1000 ppm, vorzugsweise weniger als etwa 750 ppm, weiter bevorzugt weniger als etwa 500 ppm, besonders bevorzugt weniger als etwa 300 ppm zur Herstellung eines Katalysators.

**26.** Verwendung eines Titandioxids gemäß Anspruch 25, wobei der Gehalt des $TiO_2$ an Phosphor, berechnet als elementarer Phosphor, weniger als etwa 300 ppm, insbesondere weniger als etwa 280 ppm beträgt, und vorzugsweise der Gehalt des $TiO_2$ an Niob, berechnet als Nb, mehr als etwa 500 ppm, insbesondere mehr als etwa 1000 ppm beträgt.

**27.** Verwendung eines Titandioxids gemäß Anspruch 25 oder 26 zur Herstellung eines Katalysators zur Gasphasenoxidation von Kohlenwasserstoffen, insbesondere zur Herstellung von Phthalsäureanhydrid durch Gasphasenoxidation von o-Xylol, Naphthalin oder Gemischen davon, zur Methanoloxidation zu Formaldehyd, zur oxidativen Dehydrierung von Alkanen, zur partiellen Oxidation von Aldehyden oder Alkoholen zu den entsprechenden Carbonsäuren.

**28.** Verwendung eines Titandioxids gemäß einem der Ansprüche 25 bis 26 zur Herstellung eines Katalysators zur Gasphasenoxidationen von aromatischen Kohlenwasserstoffen wie Benzol, Xylolen, Naphthalin, Toluol oder Durol zur Herstellung von Carbonsäuren und/oder Carbonsäureanhydriden; bei der Ammoxidation von Alkanen und Alkenen, der Ammoxidation von Alklyaromaten und Alkylheteroaromaten zu den entsprechenden Cyanoverbindungen, insbesondere die Ammoxidation von 3-Methylpyridin (b-Picolin) zu 3-Cyanopyridin, bei der Oxidation von 3-Methylpyridin zu Nicotinsäure, bei der Oxidation von Acenaphthen zu Naphthalsäureanhydrid, oder von Duren zu Pyromellitsäureanhydrid; bei der Herstellung von Naphthalsäureanhydrid aus Acenaphthen sowie der Herstellung von Cyanopyridin aus Alkylpyridin (Picolin) durch Ammoxidation, wie beispielsweise der Umwandlung von 3-Methylpyridin zu 3-Cyanopyridin.

**Europäisches Patentamt**

**EUROPÄISCHER RECHERCHENBERICHT**

Nummer der Anmeldung

EP 06 01 0681

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 03/018481 A (SHELL INT RESEARCH [NL]; CHETTOUF ABDERRAHMANE [NL]; MESTERS CAROLUS M) 6. März 2003 (2003-03-06) * Beispiele; Tabellen * * Seite 9, Zeile 7 - Zeile 10 * * Seite 8, Zeile 20 - Seite 9, Zeile 7 * * Ansprüche 1,9 * ----- | 1-23,25 | INV. C07C51/265 C07C51/31 B01J21/06 |
| X | US 5 527 469 A (LAWHORNE EARL R [US] ET AL) 18. Juni 1996 (1996-06-18) * Spalte 3, Zeile 55 - Zeile 61 * * Anspruch 1; Beispiele; Tabelle 1 * * Abbildung 1 * * Spalte 4, Zeile 54 - Zeile 57 * ----- | 1-23,25 | |
| D,A | DE 198 23 262 A1 (BASF AG [DE]) 2. Dezember 1999 (1999-12-02) * Anspruch 1; Beispiele * ----- | | |
| D,A | DE 198 23 275 A1 (BASF AG [DE]) 2. Dezember 1999 (1999-12-02) * Anspruch 1; Beispiele * ----- | | |

RECHERCHIERTE SACHGEBIETE (IPC)

C07C
B01J

Der vorliegende Recherchenbericht wurde für alle Patentansprüche erstellt

| Recherchenort | Abschlußdatum der Recherche | Prüfer |
|---|---|---|
| München | 31. Oktober 2006 | Holzwarth, Arnold |

**ANHANG ZUM EUROPÄISCHEN RECHERCHENBERICHT**
**ÜBER DIE EUROPÄISCHE PATENTANMELDUNG NR.**

EP 06 01 0681

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-10-2006

| Im Recherchenbericht angeführtes Patentdokument | Datum der Veröffentlichung | Mitglied(er) der Patentfamilie | Datum der Veröffentlichung |
|---|---|---|---|
| WO 03018481 A | 06-03-2003 | CA 2458028 A1<br>MX PA04001404 A<br>US 2004179995 A1<br>ZA 200400954 A | 06-03-2003<br>27-05-2004<br>16-09-2004<br>20-10-2004 |
| US 5527469 A | 18-06-1996 | EP 0748769 A1 | 18-12-1996 |
| DE 19823262 A1 | 02-12-1999 | CN 1131859 C<br>WO 9961433 A1<br>EP 1084115 A1<br>ES 2197684 T3<br>ID 27092 A<br>JP 2002516319 T<br>TW 444004 B<br>US 6700000 B1 | 24-12-2003<br>02-12-1999<br>21-03-2001<br>01-01-2004<br>01-03-2001<br>04-06-2002<br>01-07-2001<br>02-03-2004 |
| DE 19823275 A1 | 02-12-1999 | CN 1302294 A<br>WO 9961434 A1<br>EP 1082317 A1<br>ID 26586 A<br>JP 2002516320 T<br>TW 487705 B<br>US 6362345 B1 | 04-07-2001<br>02-12-1999<br>14-03-2001<br>18-01-2001<br>04-06-2002<br>21-05-2002<br>26-03-2002 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1082317 B1
- EP 1084115 B1
- WO 200511615 A1
- EP 0964744 B1
- DE 2159441 A
- DE 1642938 A
- DE 1769998 A
- US 2035606 A
- DE 1280756
- DE 19828583
- DE 19709589
- EP 744214 A
- DE 2106796
- WO 9800778 A
- EP 714700 A
- WO 9837967 A
- WO 9961433 A
- DE 10040827 A1

**In der Beschreibung aufgeführte Nicht-Patentliteratur**

- **SAURAMBAEVA ; SEMBAEV.** *Eurasian ChemTech Journal,* 2003, vol. 5, 267-270
- **R. CHUCK.** *Applied Catalysis, A: General,* 2005, vol. 280 (1), 75-82
- Phtalic Acid and Derivatives. **K. TOWAE ; W. ENKE ; R. JÄCKH ; N. BHARGANA.** Ullmann's Encyclopedia of Industrial Chemistry. 1992, vol. A. 20, 181
- *J. Am. Chem. Soc.,* 1938, vol. 60, 309